Europäisches Patentamt

European Patent Office

Office européen des brevets

(11) Veröffentlichungsnummer: **0 422 428 A2**

## (12) EUROPÄISCHE PATENTANMELDUNG

(21) Anmeldenummer: **90118154.5**

(22) Anmeldetag: **21.09.90**

(51) Int. Cl.5: **G01N 1/10**

(30) Priorität: **10.10.89 DE 3933754**

(43) Veröffentlichungstag der Anmeldung:
**17.04.91 Patentblatt 91/16**

(84) Benannte Vertragsstaaten:
**AT BE CH DE DK ES FR GB GR IT LI LU NL SE**

(71) Anmelder: **SCHWARTE-WERK GMBH**
**Am Industriepark 2-10**
**W-2059 Büchen(DE)**

(72) Erfinder: **Pust, Manfred, Ing. grad.**
**Wagenfeldstrasse 52**
**W-4722 Enningerloh(DE)**

(54) **Vorrichtung zur Abgabe von Flüssigkeiten.**

(57) Die Erfindung bezieht sich auf eine Vorrichtung zur Abgabe von Flüssigkeitsproben, insbesondere einer Vielzahl nacheinander gewonnener Milchproben, in entsprechend viele Probegefäße, die vorzugsweise mit einem Deckel aus einem sich nach Durchstechen selbst schließenden Material verschlossen sind, wobei eine Injektionskanüle mit zwei Strömungswegen, gebildet aus parallelen Verbindungswegen in einem inneren und zwischen diesem und einem äußeren Rohr, vorgesehen ist.

Durch die Erfindung wird unter anderem ein Nachtropfen der Injektionskanüle sicher vermieden.

Dies wird u.a. dadurch erreicht, daß während eines Einstechhubes der Injektionskanüle (K) der erste und der zweite Strömungsweg (3a bzw. 11) jeweils an ihrem probegefäßseitigen Ende gegenüber der Umgebung der Injektionskanüle geschlossen sind, und daß sich die Strömungswege gegenüber ihrer Umgebung am Ende des Einstechhubes infolge Relativbewegung der die Strömungswege bildenden Berandungen (3, 5) gegenseitig öffnen und durch Hubrichtungsumkehr gegenseitig verschließen.

Fig. 3

Xerox Copy Centre

# VORRICHTUNG ZUR ABGABE VON FLÜSSIGKEITSPROBEN

Die Erfindung betrifft eine Vorrichtung zur Abgabe von Flüssigkeitsproben, insbesondere einer Vielzahl nacheinander gewonnener Milchproben in entsprechend viele Probegefäße, die vorzugsweise mit einem Deckel aus einem sich nach Durchstechen selbst schließenden Material verschlossen sind, mit einer Injektionskanüle, die parallele Verbindungswege in einem inneren und zwischen diesem und einem äußeren Rohr aufweist, wobei der Verbindungsweg im inneren Rohr einen ersten Strömungsweg für die in das Probegefäß zu überführende Flüssigkeitsprobe und der andere Verbindungsweg einen zweiten Strömungsweg für das aus dem Probegefäß verdrängte Gas bildet, und wobei die Strömungswege in einer Spülstellung der Vorrichtung vor Abgabe der Flüssigkeisprobe mit der Probenflüssigkeit durchspült werden.

Eine Vorrichtung der einleitend gekennzeichneten Gattung ist aus der DE 34 48 178 A1 bekannt. Diese bekannte Vorrichtung (vergleiche Fig. 3a) zeichnet sich gegenüber einer weiteren bekannten und in ihrer Funktion vergleichbaren Probeabgabevorrichtung (DE-AS 27 12 208) dadurch aus, daß in einer Spülstellung (vergleiche Fig. 3a) die beiden Strömungswege der Injektionskanüle mit der Probenflüssigkeit durchspült werden können, so daß alle mit der Probemenge des vorherigen Lieferanten benetzten Flächen vollständig gereinigt werden. Demgegenüber kann bei der Probeabgabevorrichtung gemäß DE-AS 27 12 208 lediglich die Füllnadel 3 in der Spülstellung mit der Probeflüssigkeit durchspült werden (vergleiche Fig. I), nicht jedoch das Röhrchen 18, das in der Probeabgabestellung (vergleiche Fig. II) unter Umständen mit der Probeflüssigkeit kontaminiert wird.

Da der Spülvorgang vor der Abgabe der Probeflüssigkeit in das Probegefäß stattfinden muß, damit die Flüssigkeitsreste des vorhergehenden Lieferanten aus den Strömungswegen entfernt werden können, ist die Injektionskanüle nach diesem Spülvorgang mit der Probeflüssigkeit des aktuellen Lieferanten vollständig befüllt. Es besteht nun bei allen bekannten Probeabgabevorrichtungen mehr oder weniger die Gefahr, daß aus der Injektionskanüle auf ihrem Weg von der Spülstellung in die Probeabgabestellung, bei der sich zumindest ihre Spitze innerhalb des Probegefäßes befindet, Probeflüssigkeit heraustropft und sich auf dem Deckel des zur Probeabfüllung bereitstehenden Probegefäßes niederschlägt. Da eine Vielzahl von Probegefäßen dicht bei dicht in einem sogenannten Magazin angeordnet sind, ist nicht auszuschließen, daß die aus der Injektionskanüle nachtropfende Probeflüssigkeit auch auf Deckel der benachbarten Probegefäße verteilt wird. Eine derartige Kontamination ist einerseits aus Gründen einer hygienischen und verschleppungsfreien Probenüberführung in das Probegefäß und andererseits unter rein optischen Gesichtspunkten nicht hinzunehmen.

Der vorliegenden Erfindung liegt daher die Aufgabe zugrunde, eine Vorrichtung der einleitend gekennzeichneten Gattung derart weiterzubilden, daß ein Nachtropfen der Injektionskanüle sicher vermieden wird.

Diese Aufgabe wird durch die Kennzeichenmerkmale des Anspruchs 1 gelöst. Vorteilhafte Ausführungsformen der Vorrichtung gemäß der Erfindung sind Gegenstand der abhängigen Ansprüche 2 bis 9.

Erst wenn die Injektionskanüle den Deckel des Probegefäßes durchstochen hat, wird der Strömungsweg für die Probeflüssigkeit einerseits und der Strömungsweg für das aus dem Probegefäße verdrängte Gas andererseits geöffnet. Das Öffnen und Verschließen der Strömungswege erfolgt dabei durch Relativbewegung der die Strömungswege bildenden Berandungen und zwar ohne daß ein weiteres Verschlußteil notwendig ist. Während des Einstechvorganges und des zeitlich vorgeordneten Spülvorganges der Injektionskanüle sind die Strömungswege gegenüber der Umgebung geschlossen. Ein Herausziehen der Injektionskanüle aus dem Probegefäß ist erst nach Verschluß der Strömungswege möglich.

Eine vorteilhafte Ausführungsform der Vorrichtung gemäß der Erfindung zeichnet sich dadurch aus, daß das den ersten Strömungsweg bildende innere Rohr an seinem Ende durch eine Nadel verschlossen wird, wobei oberhalb derselben eine Austrittsöffnung des ersten Strömungsweges vorgesehen ist. Das gegenseitige Öffnen und Schließen der Strömungswege erfolgt dadurch, daß sich das stirnseitige Ende des äußeren Rohres an der über den Außendurchmesser des inneren Rohres radial überstehenden Nadel anlegt. In diesem Zusammenhang erweist es sich weiterhin als vorteilhaft, die Nadel austauschbar zu gestalten, wobei vorgesehen ist, daß sie aus einer Nadelspitze, einem Nadelkopf und einem in den Innendurchgang des inneren Rohres eingreifenden Nadelschaft besteht. Das äußere und das innere Rohr sind relativ zueinander beweglich; ihre gegenseitige Anpressung in der Schließstellung wird durch Federvorspannung bewirkt.

Das Einstechen der Injektionskanüle in den Deckel des Probegefäßes gestaltet sich problemlos und ohne über das notwendige Maß einer Trennung und Aufweitung des Stopfenmaterials hinausgehend, wenn, wie dies eine weitere Ausführungsform der Vorrichtung gemäß der Erfindung vorsieht, der Nadelkopf und das äußere Rohr in ihrer Schließstellung miteinander an ihrer äußeren Mantelfläche bündig zueinander abschließen.

Ein absolut dichter Verschluß der Injektionskanüle wird nach einer weiteren vorteilhaften Ausführungsform der Vorrichtung gemäß der Erfindung durch eine Dichtung sichergestellt, die zwischen Nadel bzw. Nadelkopf und der Stirnseite des äußeren Rohres vorgesehen ist. Darüber hinaus ist von Vorteil, wenn die Dichtung außenseits einerseits mit der Nadel bzw. dem Nadelkopf und andererseits mit dem äußeren Rohr bündig abschließt. In der Schließstellung der Injektionskanüle ist eine vollständige Durchspülung der beiden Strömungswege mit Probeflüssigkeit möglich, wenn, wie dies eine weitere vorteilhafte Ausgestaltung der Vorrichtung gemäß der Erfindung vorsieht, die Strömungswege über die in der Wandung des inneren Rohres angeordnete Austrittsöffnung miteinander verbunden sind.

Solange der Antrieb der Injektionskanüle in ihrer Einstechrichtung mit Druckmittel beaufschlagt und das äußere Rohr in seiner unteren Lage positioniert ist, bleibt die Injektionskanüle unter der von dem Druckmittel ausgeübten Antriebskraft geöffnet. Sobald der Antrieb nicht mehr in der vorstehenden Weise mit Druckmittel beaufschlagt wird, stellt eine Feder, wie dies eine weitere vorteilhafte Ausgestaltung der Vorrichtung gemäß der Erfindung vorsieht, den Verschluß der Injektionskanüle sicher. Die Feder ist dabei derart angeordnet, daß sie bei Umsteuerung des Druckmittels zwecks Einleitung der Hubrichtungsumkehr der Injektionskanüle zwangsläufig die Relativbewegung des inneren und des äußeren Rohres zueinander bis zum gegenseitigen Verschluß bewirkt.

Eine weitere vorteilhafte Ausführungsform der Vorrichtung gemäß der Erfindung sieht vor, daß der erste Strömungsweg unmittelbar mit einem Zuführungsrohr verbunden ist, welches in eine Ausnehmung eines Stößels in Hubrichtung der Vorrichtung verschieblich ist. Dadurch wird die Anzahl der Dichtungen im flüssigkeitsbeaufschlagten System auf die notwendige Mindestanzahl begrenzt.

Ein Ausführungsbeispiel der Erfindung ist anhand von Zeichnungen dargestellt und wird im folgenden näher beschrieben. Es zeigen

Fig. 1 einen Querschnitt durch die erfindungsgemäße Vorrichtung in ihrer oberen Endlage;

Fig. 2 die Vorrichtung gemäß Fig. 1 in ihrer unteren Endlage, wobei die Injektionskanüle nach Durchstechen des Deckels des Probegefäßes noch geschlossen ist und

Fig. 3 die Probeabgabestellung der Vorrichtung gemäß den Figuren 1 und 2 mit der geöffneten Injektionskanüle.

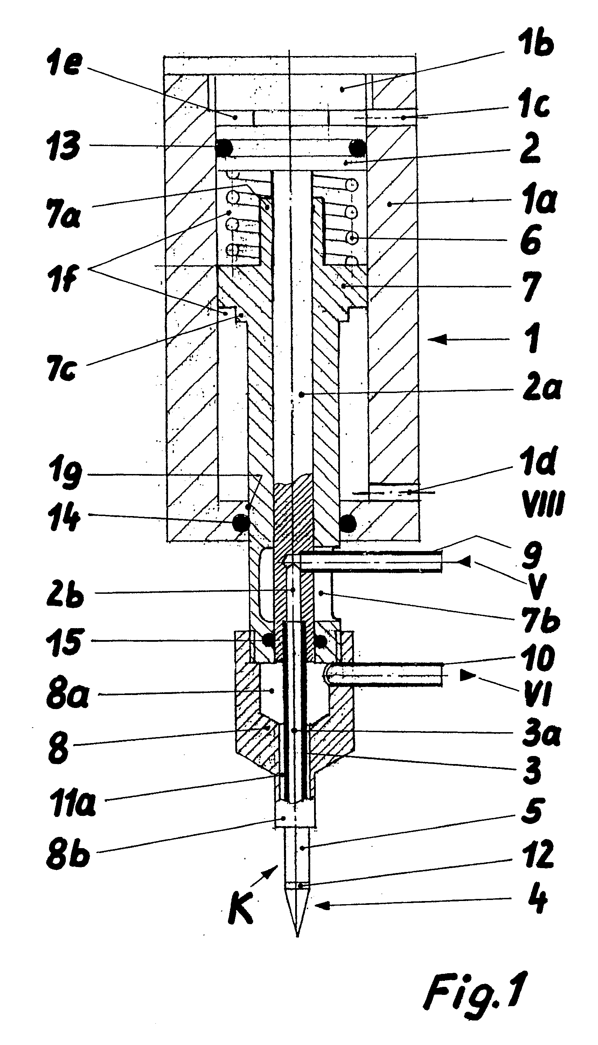

Die Vorrichtung zur Abgabe von Flüssigkeitsproben besteht gemäß Fig. 1 aus einem Antrieb 1, welcher in einem Gehäuse 1a einen oberen und einen unteren Antriebsraum 1e bzw. 1f beherbergt. Der obere Antriebsraum 1e wird einerseits durch ein Verschlußteil 1b und andererseits durch einen Antriebskolben 2 begrenzt. Der untere Antriebsraum 1f wird durch die Unterseite des Antriebskolbens 2 und die Wandung des Gehäuses 1a gebildet, wobei ein mit Spiel innerhalb des Gehäuses 1a geführter Stößel 7 aus letzterem über eine Bohrung 1g und mittels einer Gehäusedichtung 14 abgedichtet nach unten herausgeführt ist. Der obere Antriebsraum 1e weist einen ersten Druckmittelanschluß 1c und der untere Antriebsraum 1f einen zweiten Druckmittelanschluß 1d auf. Zwischen dem Antriebskolben 2 und dem Stößel 7 ist eine Feder 6 vorgesehen. Eine Kolbenstange 2a ist einerseits mit dem Kolben 2 verbunden und durchdringt andererseits konzentrisch den Stößel 7. Letzterer und die Kolbenstange 2a sind an ihrem unteren Ende über eine Stößeldichtung 15 gegeneinander abgedichtet. Die Kolbenstange 2a ist an ihrem Ende mit einem inneren Rohr 3 verbunden, dessen Innendurchgang 3a sich in einer Zuführbohrung 2b innerhalb der Kolbenstange 2a ein Stück nach oben fortsetzt und mit einem Zuführungsrohr 9, welches von der Seite her an die Kolbenstange 2a angeschlossen ist, eine Verbindung aufweist. Eine Ausnehmung 7b im Stößel 7 im Bereich des Zuführrohres 9 stellt sicher, daß letzteres infolge der Relativbewegung zwischen der Kolbenstange 2a und dem Stößel 7 in Hubrichtung der Vorrichtung verschieblich ist.

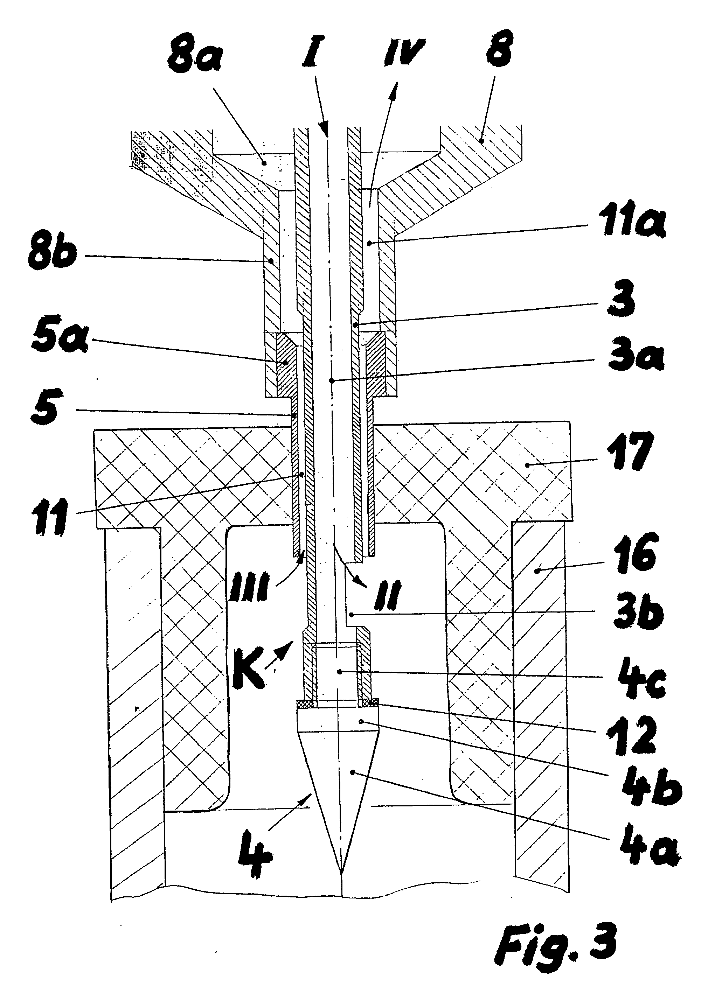

Am unteren Ende des Stößels 7 ist eine Hülse 8 vorgesehen, die innenseits eine Kammer 8a bildet und an ihrem unteren Ende sich in ein Aufnahmeteil 8b verjüngt (vergleiche hierzu auch Fig.3). Ein äußeres Rohr 5 greift mit einem Befestigungsteil 5a in das Aufnahmeteil des 8b von innen her ein. Das innere Rohr 3 ist durch das äußere Rohr 5 konzentrisch hindurchgeführt und bildet mit diesem den Ringspalt 11a, der Teil des zweiten Strömungsweges darstellt.

Eine Kolbendichtung 13 sorgt für eine einwandfreie Abdichtung des Kolbens 2 innerhalb des Gehäuses 1a, wobei allein diese Dichtung die Trennung des oberen und des unteren Antriebsraumes 1e bzw. 1f voneinander sicherstellt. Der Stößel 7 verfügt über so viel radiales Spiel innerhalb des Gehäuses 1a, daß ein Durchtritt des über den zweiten Druckmittelanschluß 1d zugeführten Druckmittels bis zur Unterseite des Antriebskolbens 2 gegeben ist.

Wenn der Antrieb 1 über den ersten Druckmittelanschluß 1c mit Druckmittel beaufschlagt wird, erfolgt eine abwärts gerichtete Verschiebung des Antriebskolbens 2, der über die unter Vorspannung stehende Feder 6 den Stößel 7 ebenfalls nach unten mitnimmt. Sobald sich der Stößel 7 über einen unteren Anschlag 7c an der stirnseitigen Begrenzung des Gehäuses 1a anlegt, kann der Antriebskolben 2 eine weitere gegen den Stößel 7 gerichtete Abwärtsbewegung durchführen, bis er an einem oberen Anschlag 7a des Stößels 7 zur Anlage kommt. Die vorgenannte Relativbewegung des Antriebskolbens 2 und des Stößels 7 ergibt die nachfolgend noch genauer beschriebene Öffnungsbewegung der Injektionskanüle K bzw. in umgekehrter Bewegungsrichtung ihre entsprechende Schließbewegung.

Die Kammer 8a ist über ein Abführungsrohr 10 mit der Umgebung der Vorrichtung verbunden. Der Ringspalt 11a, die Kammer 8a und das Abführungsrohr 10 bilden einen Teil des zweiten Strömungsweges, über den in der Spülstellung der Vorrichtung, die in Figur 1 gegeben ist, die Spülflüssigkeit geführt wird. Mit VI ist der Spülflüssigkeitsaustritt und mit V ist an dem Zuführungsrohr 9 der diesbezügliche Spülflüssigkeitseintritt gekennzeichnet. Um sicherzustellen, daß der Antrieb in seiner oberen Endlage verbleibt, wird dem unteren Antriebsraum 1f das Druckmittel VIII zugeführt. Die Injektionskanüle K weist an ihrem Ende eine Nadel 4 und eine Dichtung 12 auf. Aufbau und Funktion hierzu sind in der Beschreibung zu Figur 3 detailliert enthalten.

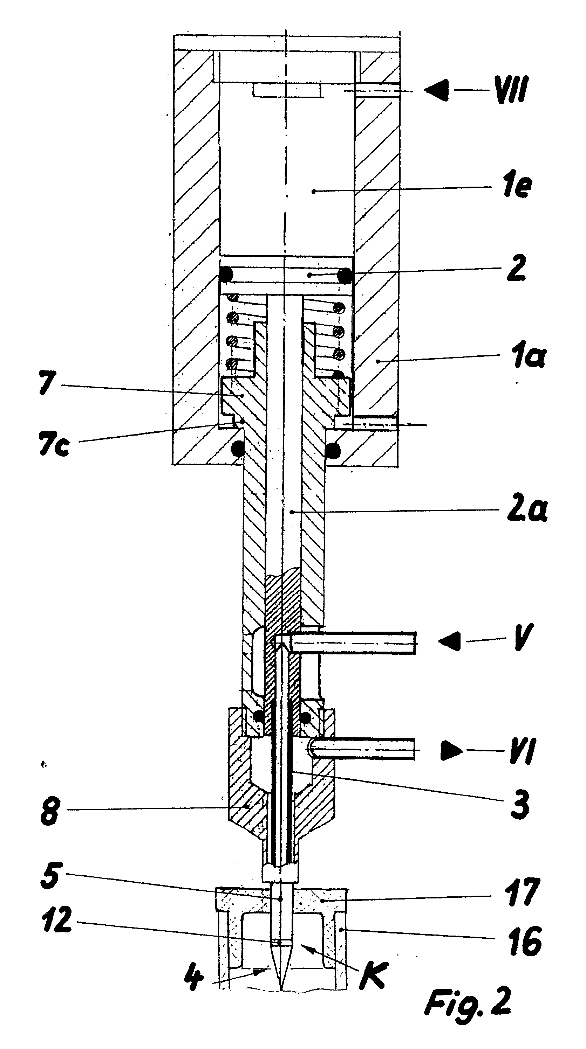

Figur 2 zeigt die Vorrichtung zur Abgabe von Flüssigkeitsproben in ihrer unteren Endlage, wobei die Injektionskanüle K gegenüber einem Probegefäß 16, dessen Deckel 17 sie durchstoßen hat, noch geschlossen ist. Eine weitere Ansteuerung des oberen Antriebsraumes 1e mit einem Druckmittel VII wird nachfolgend die weitere Abwärtsbewegung des Antriebskolbens 2, der Kolbenstange 2a, des inneren Rohres 3 und der mit diesem verbundenen Nadel 4 bewirken, so daß eine Trennung des äußeren Rohres 5 von der Nadel 4, mit der die Dichtung 12 formschlüssig verbunden ist, stattfindet. Der Stößel 7 verharrt, da er über seinen unteren Anschlag 7c am Gehäuse 1a anliegt, in Verbindung mit der Hülse 8 in der dargestellten Endlage, bis eine Umsteuerung des Antriebes 1 eingeleitet wird. Selbst in der dargestellten unteren Endlage der Vorrichtung ist noch ein Durchspülen der Injektionskanüle K möglich, ohne daß ein Austritt der Spülflüssigkeit in das Probegefäß 16 stattfinden kann. Der Spülflüssigkeitseintritt ist mit V, sein Austritt ist mit VI gekennzeichnet.

Figur 3 zeigt die Probeabgabestellung der erfindungsgemäßen Vorrichtung im Bereich des unteren Endes der Injektionskanüle K nach Durchstechen des Deckels 17 des Probegefäßes 16. Auf das äußere Rohr 5, welches mit seinem Befestigungungsteil 5a innenseits im Aufnahmeteil 8b der Hülse 8 angeordnet ist, wurde bereits in der Beschreibung zu Figur 1 Bezug genommen. Ebenso wurde bereits erwähnt, daß das innere Rohr 3 konzentrisch durch das äußere Rohr 5 hindurchgeführt ist und mit diesem einen Ringspalt 11, der Teil des zweiten Strömungsweges ist, bildet. Die Nadel 4 besteht aus einer Nadelspitze 4a, einem Nadelkopf 4b und einem Nadelschaft 4c. Letzterer greift in den Innendurchgang 3a des inneren Rohres 3 ein und bildet dadurch dessen stirnseitigen Verschluß. Zwischen dem Nadelkopf 4b und der Stirnseite des inneren Rohres 3 ist die Dichtung 12 angeordnet. Sowohl diese als auch der Nadelkopf 4b haben einen radialen Überstand gegenüber der äußeren Abmessung des inneren Rohres 3, so daß ein abdichtender Anschlag für die Stirnseite des äußeren Rohres 5 gegeben ist. Oberhalb des Nadelkopfes 4b ist in der Wandung des inneren Rohres 3 eine Austrittsöffnung 3b vorgesehen, die in der gezeichneten Stellung der Injektionskanüle K eine Verbindung zwischen dem Innendurchgang 3a und dem Raum innerhalb des Probegefäßes 16 herstellt (erster Strömungsweg).

Der zweite Strömungsweg, dessen letzter Teil durch den Ringspalt 11 zwischen dem äußeren Rohr 5 und dem inneren Rohr 3 gebildet wird, ist ebenfalls zum Innenraum des Probegefäßes 16 hin offen. Mit I ist die über den ersten Strömungsweg 3a zugeführte Probeflüssigkeit gekennzeichnet. Diese tritt bei II über die Austrittsöffnung 3b in das Probengefäß 16 aus. Im Zuge der Abgabe der Flüssigkeitsprobe in das Probegefäß 16 wird ein entsprechendes Gasvolumen aus letzterem verdrängt. Dieses Gas kann an der mit III gekennzeichneten Stelle in den Ringspalt 11 eintreten und gelangt über den sich anschließenden weiteren Ringspalt 11a an der mit IV gekennzeichneten Stelle in die Kammer 8a, von wo aus es über das nicht dargestellte Abführungsrohr 10 in die Umgebung der Vorrichtung fortgeleitet wird.

Sobald der Antrieb 1 (vergleiche Fig. 1) umgesteuert wird, bewirkt die Feder 6 eine Relativbewegung zwischen dem äußeren Rohr 5 und dem inneren Rohr 3 dergestalt, daß die Dichtung 12 an das stirnseitige Ende des äußeren Rohres 5 dichtend herangeführt wird. Die Injektionskanüle K ist damit gegenüber dem Innenraum des Probegefäßes 16 verschlossen. Zwischen dem ersten und dem zweiten Strömungsweg 3a bzw. 11 besteht in dieser Schließstellung der Injektionskanüle K nach wie vor eine Verbindung über die Austrittsöffnung 3b, so daß, beginnend mit dieser Schließstellung, ein Durchspülen der Injektionskanüle K möglich ist.

Der Nadelkopf 4b und das äußere Rohr 5 schließen in ihrer Schließstellung miteinander an ihrer äußeren Mantelfläche bündig zueinander ab. Die Dichtung ist dabei außenseits so bemessen, daß sie sich einerseits übergangslos an den Nadelkopf 4b und andererseits an das äußere Rohr 5 anschließt. Dadurch ist das Einstechen in den und das Herausziehen aus dem Deckel 17 des Probegefäßes 16 ohne seine über das notwendige Maß hinausgehende Beschädigung möglich.

Durch das sichere Verschließen der Injektionskanüle K im Anschluß an den Spülvorgang und im Vorwege der Abgabe der Flüssigkeitsprobe ist es nicht mehr erforderlich, wie dies bei den bekannten Vorrichtungen zwingend gegeben ist, das Kanülensystem in dieser Phase völlig drucklos zu schalten, damit ein Verspritzen und Nachtropfen der dort anstehenden Flüssigkeitsprobe unterbunden wird. Die erfindungsgemäße Vorrichtung reduziert somit auch den Aufwand an bislang notwendigen Schaltgliedern und Steuerungsfunktionen.

## Ansprüche

1. Vorrichtung zur Abgabe von Flüssigkeitsproben, insbesondere einer Vielzahl nacheinander gewonnener Milchproben, in entsprechend viele Probegefäße, die vorzugsweise mit einem Deckel aus einem sich nach Durchstechen selbst schließenden Material verschlossen sind, mit einer Injektionskanüle, die parallele Verbindungswege in einem inneren und zwischen diesem und einem äußeren Rohr aufweist, wobei der Verbindungsweg im inneren Rohr einen ersten Strömungsweg für die in das Probegefäß zu überführende Flüssigkeitsprobe und der andere Verbindungsweg einen zweiten Strömungsweg für das aus dem Probegefäß verdrängte Gas bildet, und wobei die Strömungswege in einer Spülstellung der Vorrichtung vor Abgabe der Flüssigkeitsprobe mit der Probenflüssigkeit durchspült werden, **dadurch gekennzeichnet,** daß während eines Einstechhubes der Injektionskanüle (K) der erste und der zweite Strömungsweg (3a bzw. 11) jeweils an ihrem probegefäßseitigen Ende gegenüber der Umgebung der Injektionskanüle geschlossen sind, und daß sich die Strömungswege gegenüber ihrer Umgebung am Ende des Einstechhubes infolge Relativbewegung der die Strömungswege bildenden Berandungen (3, 5) gegenseitig öffnen und durch Hubrichtungsumkehr gegenseitig verschließen.

2. Vorrichtung nach Anspruch 1, **dadurch gekennzeichnet,** daß das innere Rohr (3) an seinem Ende durch eine Nadel (4) verschlossen ist, daß oberhalb der Nadel (4) eine Austrittsöffnung des ersten Strömungsweges (3a) vorgesehen ist, und daß der zweite Strömungsweg (11) gegenüber der Umgebung durch Anlage des stirnseitigen Endes des äußeren Rohres (5) an der Nadel (4) geschlossen ist.

3. Vorrichtung nach Anspruch 2, **dadurch gekennzeichnet,** daß die Nadel (4) austauschbar ist und aus einer Nadelspitze (4a), einem Nadelkopf (4b) und einem in den Innendurchgang des inneren Rohres (3) eingreifenden Nadelschaft (4c) besteht.

4. Vorrichtung nach Anspruch 2 oder 3, **dadurch gekennzeichnet,** daß die Nadel bzw. der Nadelkopf (4b) und das äußere Rohr (5) in ihrer Schließstellung miteinander an ihrer äußeren Mantelfläche bündig zueinander abschließen.

5. Vorrichtung nach einem der Ansprüche 1 bis 4, **dadurch gekennzeichnet,** daß zwischen der Nadel (4) bzw. dem Nadelkopf (4b) und der Stirnseite des äußeren Rohres (5) eine Dichtung (12) vorgesehen ist.

6. Vorrichtung nach Anspruch 5, **dadurch gekennzeichnet,** daß die Dichtung (12) außenseits einerseits mit dem Nadelkopf (4b) und andererseits mit dem äußeren Rohr (5) bündig abschließt.

7. Vorrichtung nach einem der Ansprüche 1 bis 6, **dadurch gekennzeichnet,** daß die Strömungswege (3a, 11) in der Schließstellung der Injektionskanüle (K) über die Austrittsöffnung (3b) miteinander verbunden sind.

8. Vorrichtung nach einem der Ansprüche 1 bis 7, **dadurch gekennzeichnet,** daß der Verschluß der die Strömungswege (3a, 11) bildenden Berandungen (3, 5) durch Vorspannung einer Feder (6) zwischen einem mit der Berandung (3) verbundenen Antriebskolben (2) und einem mit der Berandung (5) verbundenen Stößel (7) sichergestellt ist.

9. Vorrichtung nach Anspruch 8, **dadurch gekennzeichnet** daß der erste Strömungsweg (3a) unmittelbar mit einem Zuführungsrohr (9) verbunden ist, welches in einer Ausnehmung (7b) des Stößels 7 in Hubrichtung der Vorrichtung verschieblich ist.

Fig.1

VII

1e

2

1a

7

7c

2a

V

VI

3

8

5

17

12

16

4

K

Fig. 2

Fig. 3